# EUROPÄISCHE PATENTSCHRIFT

(11) **EP 1 259 272 B1**
(45) Veröffentlichungstag und Bekanntmachung des Hinweises auf die Patenterteilung: **07.04.2004**
(21) Anmeldenummer: 01902235.9
(22) Anmeldetag: 13.02.2001
(51) Int. Cl.: A61M 5/14, A61M 25/00, A61M 25/06

(54) **MIKROPERFUSIONSVORRICHTUNG**
MICROPERFUSION DEVICE
DISPOSITIF DE MICROPERFUSION

(30) Priorität: 25.02.2000 DE 10008825
(43) Veröffentlichungstag der Anmeldung: 27.11.2002
(73) Patentinhaber: Disetronic Licensing AG, 3401 Burgdorf (CH)
(72) Erfinder: DIERMANN, Ulrich, CH-2563 Ipsach (CH); HAUETER, Ulrich, CH-3506 Grosshöchstetten (CH)
(74) Vertreter: Schwabe - Sandmair - Marx
(86) Internationale Anmeldenummer: PCT/CH2001/000095
(87) Internationale Veröffentlichungsnummer: WO 2001/062316

(56) Entgegenhaltungen:
- WO-A-88/05643
- WO-A-92/02270

## Beschreibung

Die Erfindung betrifft eine Mikroperfusionsvorrichtung zur Gewinnung wenigstens eines Inhaltsstoffs einer Körperflüssigkeit mittels eines subkutan plazierten Perfusionskatheters. Eine derartige Mikroperfusionsvorrichtung gemäß dem Oberbegriff des Anspruchs 1 ist aus der WO 92/02270 A bekannt.

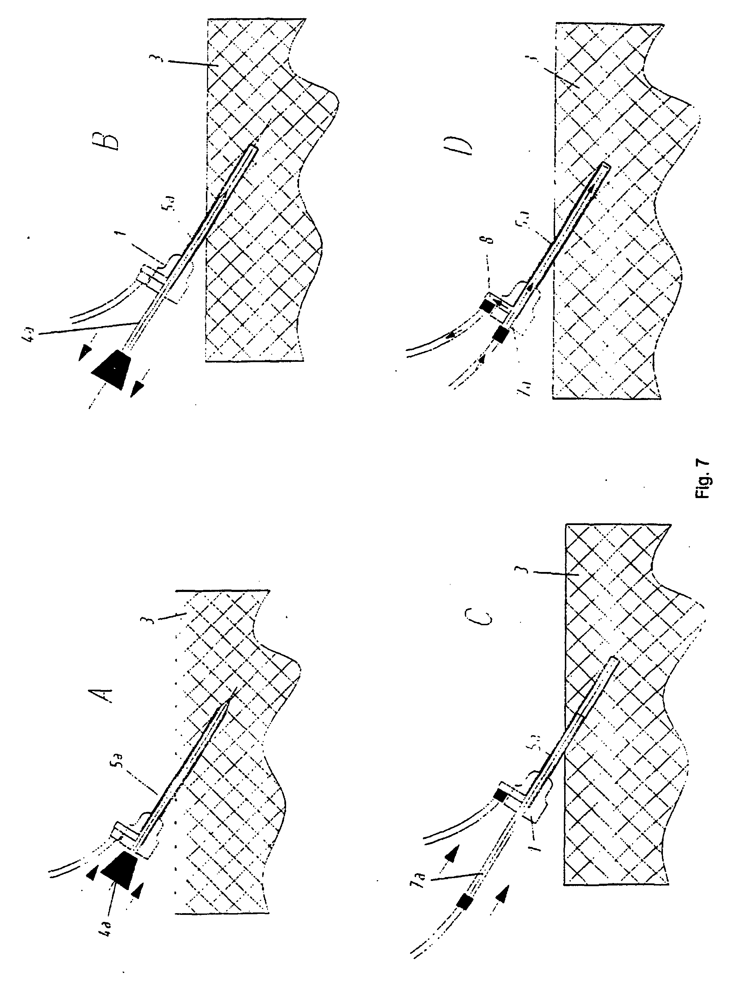

Figur 7D zeigt eine Mikroperfusionsvorrichtung, wie sie dem Prinzip nach beispielsweise aus "Open-Flow Microperfusion ...", Z. Trajanoski et al., Diabetes Care, Vol. 20, No. 7, Juli 1997, Seiten 1114 ff., bekannt ist. Die Vorrichtung weist ein Gehäuse 1 auf, das auf der Haut befestigbar ist. Von dem Gehäuse 1' ragt ein flexibler, durchlässiger Perfusionskatheter 5a ab, der in einem Gewebe 3 subkutan plaziert ist. In den Perfusionskatheter 5a ragt ein Zuführkatheter 7a für eine Spülflüssigkeit - im folgenden Perfusat genannt - hinein. Der Perfusionskatheter 5a und der Zuführkatheter 7a bilden in dem Gewebe 3 einen doppellumigen Katheter mit einem inneren Lumen innerhalb des Zuführkatheters 7a und einem umgebenden, äußeren Lumen zwischen dem Zuführkatheter 7a und der Perfusionskatheter 5a. Das äußere Lumen mündet in dem Gehäuse 1 in einen Abführkanal 8, an den ein Abführkatheter angeschlossen ist. Zur Gewinnung von Körperflüssigkeit aus dem Gewebe 3 wird das Perfusat durch den Zuführkatheter 7a hindurch bis nahe zu einem distalen, vorderen Ende des Perfusionskatheters 5a geleitet. Nach dem Austritt des Perfusats an dem distalen, vorderen Ende des Zuführkatheters 7a strömt das Perfusat in dem äußeren Lumen an dem Zuführkatheter 7a entlang zurück. Als Perfusat wird eine Spülflüssigkeit verwendet, die ein Eindringen desjenigen Inhaltsstoffs bewirkt, dessen Konzentration in der Körperflüssigkeit ermittelt werden soll. Ferner wird durch den Spüleffekt Körperflüssigkeit durch den perforierten Perfusionskatheter 5a hindurch in das äußere Lumen gesogen. Die Mischung aus Perfusat und Körperflüssigkeit wird durch den Abführkanal 8 hindurch zu einer Messeinrichtung abgeführt. Die Fluidförderung durch die Vorrichtung hindurch erfolgt mittels einer Pumpe.

Die Vorgehensweise für eine Plazierung bis zur Perfusatgewinnung ist aus den Figuren 7A bis 7D ersichtlich. Zum Plazieren der Vorrichtung wird eine Einstechnadel 4a verwendet. Der Perfusionskatheter 5a umgibt vor dem Plazieren die Einstechnadel 4a eng anliegend. Nach dem Durchstechen der Haut, nachdem die Einstechnadel 4a in dem Gewebe 3 plaziert ist (Fig. 7A), wird die Einstechnadel 4a wieder herausgezogen (Fig. 7B), und es verbleibt der Perfusionskatheter 5a in dem Gewebe 3 in der dargestellten Stellung. Anschließend wird der Zuführkatheter 7a durch das Gehäuse 1 hindurch in den Perfusionskatheter 5a eingeführt (Fig. 7C). Er wird soweit eingeführt, dass er in dem Perfusionskatheter 5a den größten Teil der Länge des Perfusionskatheters 5a einnimmt (Fig. 7D). Das vordere Ende des Zuführkatheters 7a steht jedoch ein Stück weit hinter dem vorderen Ende des Perfusionskatheters 5a zurück.

Mikroperfusionsvorrichtungen der beschriebenen Art erfordern das Durchstechen der Haut, das Herausziehen der Einstechnadel und die nachträgliche Bereitstellung einer Perfusatzuführung.

Es ist daher eine Aufgabe der Erfindung, eine Mikroperfusionsvorrichtung zu schaffen, die auf einfache Weise plaziert und in Betrieb genommen werden kann.

Die erfindungsgemäße Mikroperfusionsvorrichtung zur Gewinnung wenigstens eines Inhaltsstoffs einer Körperflüssigkeit durch einen subkutan plazierten Perfusionskatheter ist in Anspruch 1 definiert. Sie umfasst ein Gehäuse, eine Zuführung für ein Perfusat, eine hohle Einstechnadel, den Perfusionskatheter und einen in dem Gehäuse gebildeten Abführkanal zur Abführung des Perfusats zusammen mit dem wenigstens einen Inhaltsstoff. Die Einstechnadel weist eine im subkutan plazierten Zustand distale, vordere Nadelöffnung und eine hintere Nadelöffnung auf. Die vordere Nadelöffnung wird vorzugsweise durch eine stirnseitige Öffnung in der üblichen Weise gebildet, beispielsweise durch Nadelschliff. Obgleich weniger bevorzugt, kann sie grundsätzlich jedoch auch durch eine seitliche Öffnung in einem Mantel der Einstechnadel gebildet werden. Die hintere Nadelöffnung wird in der Mantelfläche der Einstechnadel gebildet. Die Einstechnadel ist von dem Gehäuse aus einer vorderen Verschiebeposition in eine hintere Verschiebeposition verschiebbar aufgenommen. In ihrer vorderen Verschiebeposition durchragt sie den Perfusionskatether. In ihrer hinteren Verschiebeposition ragt sie in den Perfusionskatheter hinein, ihre vordere Nadelöffnung wird in der hinteren Verschiebeposition jedoch von dem Perfusionskatheter überragt. Vorzugsweise ist die Einstechnadel eine Stahlnadel. Andere Nadeln können jedoch ebenfalls Verwendung finden, solange sie eine scharfe Spitze zur sauberen Penetration der Haut haben, ausreichend starr für das Plazieren und gewebeverträglich sind.

Wie bei der beschriebenen, bekannten Mikroperfusionsvorrichtung auch, dient die Einstechnadel der Plazierung des Perfusionskatheters in einem Gewebe, dessen Körperflüssigkeit und/oder aus dessen Körperflüssigkeit der wenigstens eine Inhaltsstoff gewonnen werden soll.

Die Einstechnadel erfüllt nicht nur die Funktion der Plazierung des Perfusionskatheters, sondern erfüllt die weitere Funktion der Einleitung des Perfusats in den Perfusionskatheter. Hierzu wird durch die hintere Nadelöffnung zumindest in der hinteren Verschiebeposition der Einstechnadel eine Fluidverbindung von der Perfusatzuführung in die Einstechnadel gebildet. Im implantierten, d.h. subkutan plazierten Zustand wird somit das Perfusat durch die Perfusatzuführung, die hintere Nadelöffnung, die hohle Einstechnadel und schließlich durch deren vordere Nadelöffnung in den umgebenden Perfusionskatheter eingeleitet und spült dann den Perfusionskatheter. Indem die Einstechnadel die Doppelfunktion der Plazierung und der Einleitung des Perfusats übernimmt, entfällt das nachträgliche Einführen einer Perfusatzuführung in den Perfusionskatheter.

Vorteilhafterweise kann die erfindungsgemäße Mikroperfusionsvorrichtung durch eine recht einfache Weiterbildung von Katheterköpfen gebildet werden, wie sie beispielsweise für die Infusion von Insulin im Rahmen einer Diabetestherapie bekannt sind. Ein besonders geeigneter Katheterkopf wird durch die DE 198 21 223.4 der Anmelderin beschrieben. Der dort beschriebene Katheterkopf weist bereits eine verschiebbar im Katheterkopfgehäuse aufgenommene Einstechnadel mit einer vorderen und einer hinteren Nadelöffnung auf. Die hintere Nadelöffnung wird durch eine seitliche Öffnung gebildet, durch die hindurch nach dem Plazieren eines Infusionskatheters die Insulinlösung zum Zwecke des Primens des Katheterkopfs zugeführt wird. In Bezug auf die Einstechnadel dieses Katheterkopfs bedarf es lediglich einer fluiddichten Verbindung im Bereich der hinteren Nadelöffnung für die Zuführung des Perfusats. Ferner muss in dem Katheterkopf ein Abführkanal mit einer fluiddichten Verbindung zu dem Infusionskatheter, respektive Perfusionskatheter, ausgebildet werden. Als Perfusionskatheter ist der Infusionskatheter ferner vorzugsweise perforiert.

In einer bevorzugten Verwendung dient die erfindungsgemäße Mikroperfusionsvorrichtung der Messung bzw. Ermittlung der Glukosekonzentration in der Körperflüssigkeit in der Umgebung des implantierten Perfusionskatheters. Der wenigstens eine Inhaltsstoff der Körperflüssigkeit wird in diesem Fall durch Glukose gebildet. Vorzugsweise wird durch den Spülvorgang ganz einfach die Körperflüssigkeit zusammen mit dem Perfusat gewonnen. Es kann mittels der erfindungsgemäßen Mikroperfusionsvorrichtung bei Verwendung eines entsprechenden Perfusats bzw. Spülfluids ein bestimmter Inhaltsstoff auch selektiv zusätzlich zur mitgespülten Körperflüssigkeit gewonnen werden.

Die Perfusion erfolgt bevorzugt durch Absaugen des Abfuhrkanals mittels einer Pumpe. Das Perfusat kann aber auch in die Einstechnadel gedrückt werden. Es kann auch kombiniert eine Druck- und Saugförderung durchgeführt werden.

Erfindungsgemäß besteht die Fluidverbindung zwischen der' Perfusatzuführung und der Einstechnadel nur in der hinteren Verschiebeposition der Einstechnadel. Die Perfusatzuführung ist in diesem Fall ein in dem Gehäuse ausgebildeter Zuführkanal. In der hinteren Verschiebeposition öffnet sich die Einstechnadel mit ihrer hinteren Nadelöffnung in diesen Zuführkanal hinein. Dabei wird sichergestellt, dass in dem Gehäuse die Perfusatabführung fluiddicht gegenüber der Perfusatzuführung abgeschlossen ist. Vorzugsweise sind hierfür im Gehäuse entsprechende Dichtungsmittel vorgesehen.

Der Perfusionskatheter kann grundsätzlich durch einen mantelseitig geschlossenen Katheter mit lediglich einer offenen vorderen Stirnseite gebildet werden. Bevorzugt ist der Perfusionskatheter jedoch für den zu messenden Inhaltsstoff oder selektiv nur für den zu messenden Inhaltsstoff oder für die Körperflüssigkeit insgesamt seitlich durchlässig. Der Perfusionskatheter kann aus einem porösen Material hergestellt sein. Ist der Perfusionskatheter perforiert, so sind die seitlichen Perforationsöffnungen des Perfusionskatheters in Katheterlängsrichtung vorzugsweise langgestreckt, um eine möglichst hohe Stabilität gegen Stauchung zu erhalten. Ein Stauchen des Katheters beim Einführen in das Gewebe, auch bekannt als Peal Back Effekt, wird dadurch verhindert oder zumindest gering gehalten. Besonders bevorzugt sind die Perforationsöffnungen nicht entlang einer sich in Längsrichtung des Perfusionskatheters erstreckenden Linie, sondern in Umfangsrichtung des Perfusionskatheters zueinander versetzt bzw. auf Lücke angeordnet. Eine Perforation kann bei einer Formung des Katheters oder erst nachträglich gebildet werden, beispielsweise durch Lasern.

Um einen Perfusionskatheter zu erhalten, der möglichst schlank ist, weisen der Außenquerschnitt der Einstechnadel und der Innenquerschnitt des Perfusionskatheters eine unterschiedliche Form auf, derart, dass der Perfusionskatheter nur in Längsstreifen an der Einstechnadel anliegt und zwischen zwei benachbarten Längsstreifen ein Längsspalt verbleibt. In bevorzugten Ausführungsbeispielen weist entweder die Einstechnadel oder der Perfusionskatheter einen Querschnitt auf, der von der kreisrunden Form abweicht. Der Perfusionskatheter kann in dieser Ausbildung die Einstechnadel über deren gesamte, im Gewebe befindliche Länge eng umschmiegen. Es verbleibt zwischen der äußeren Mantelfläche der Einstechnadel und der inneren Mantelfläche des Perfusionskatheters dennoch ein Strömungsquerschnitt für das zurückströmende Perfusat. Weist beispielsweise die Einstechnadel über ihre implantierte Länge einen von der Kreisform abweichenden Außenquerschnitt auf, so kann der Perfusionskatheter einen kreisförmigen Innenquerschnitt aufweisen, der um die Nadel gespannt ist. Ebenso können der Perfusionskatheter einen nicht kreisrunden Innenquerschnitt und die Einstechnadel einen kreisrunden Außenquerschnitt aufweisen. Es können auch der Außenquerschnitt der Einstechnadel und der Innenquerschnitt des Perfusionskatheters von der kreisrunden Form abweichen, solange gewährleister ist, dass zwischen der Nadel und dem Perfusionskatheter ein zum Zwecke des Spülens ausreichender Strömungsquerschnitt verbleibt und der Perfusionskatheter die Einstechnadel zum Zwecke des sicheren Implantierens vorzugsweise eng angeschmiegt umgibt.

Vorzugsweise ist die Einstechnadel in ihrer hinteren Verschiebeposition an dem Gehäuse derart festgelegt, dass es für einen Verwender der Mikroperfusionsvorrichtung taktil spürbar ist, wenn sich die Einstechnadel in ihrer hinteren Verschiebeposition befindet. Beispielsweise kann die Einstechnadel in die hintere Verschiebeposition einfach gegen einen Anschlag bewegt werden. Die Festlegung der Einstechnadel erfolgt aber nicht nur gegen ein weiteres Verschieben über die hintere Verschiebeposition hinaus, sondern auch in Bezug auf ein Vorschieben der Einstechnadel, d.h. die Einstechnadel wird in ihrer hinteren Verschiebeposition an dem Gehäuse mittels einer Rastverbindung, vorzugsweise einer lösbaren Rastverbindung, festgelegt, wobei die hintere Nadelöffnung für die Zwecke der Rastverbindung genutzt wird.

In einer bevorzugten Ausführungsform wird die Mikroperfusionsvorrichtung nicht nur zur Gewinnung des wenigstens einen Inhaltsstoffs der Körperflüssigkeit verwendet, sondern dient gleichzeitig als Miniaturmesseinrichtung oder zumindest als Elektrodenplattform für eine Messeinrichtung. Die Messeinrichtung dient vorzugsweise der Messung bzw. Ermittlung der Konzentration des wenigstens einen Inhaltsstoffs in der Körperflüssigkeit. In der Verwendung als Elektrodenplattform ist eine Elektrode der Messeinrichtung an der Unterseite des Gehäuses, mit der das Gehäuse auf dem Gewebe aufliegt, ausgebildet. Eine Arbeitselektrode der Messeinrichtung steht mit dem abgeführten Spülfluid in elektrischem Kontakt und ist vorzugsweise in dem Abführkanal des Gehäuses angeordnet. Die an der Unterseite des Gehäuse ausgebildete Elektrode bildet die Gegenelektrode zu dieser Arbeitselektrode und dient der Messung eines elektrischen Stroms und/oder eines elektrischen Potentials. Vorzugsweise wird an der Unterseite des Gehäuses eine so große Auflagefläche gebildet, dass die Gegenelektrode eine ausreichend große Kontaktfläche zum Gewebe ausbildet und gleichzeitig als Referenzelektrode verwendet werden kann. Ferner kann sie eine Klebefunktion zum Aufkleben auf die Haut erfüllen.

In der Ausbildung als Miniaturmesseinrichtung ist in dem Gehäuse der Mikroperfusionsvorrichtung ein Sensor angeordnet, mit dem vorzugsweise die Konzentration des wenigstens einen Inhaltsstoffs in der Körperflüssigkeit gemessen wird. Genauer gesagt, es wird die Konzentration im zurückströmenden Perfusat gemessen und daraus die Konzentrationen in der Körperflüssigkeit ermittelt. Der Sensor ist vorzugsweise in einem Bereich des Abflusses angeordnet, der an das äußere Lumen angrenzt. Vorzugsweise ist er an einer Seitenwandung des Abflusses eingesetzt oder eingelassen und ragt vorzugsweise nicht aus der Wandung hervor, um den Strömungswiderstand gering zu halten. In dem zwischen dem äußeren Lumen und dem Sensor liegenden Strömungsweg sind bevorzugt keine Wandungsteile des Gehäuses oder gerade soviel Gehäusewandung angeordnet wie für eine sichere Befestigung des Sensors erforderlich ist. Der Sensor ist somit so nah wie möglich an der Probenentnahmestelle, aber außerhalb des Körpers angeordnet.

Obgleich die Ausbildung mit integriertem Sensor, die Ausbildung als Elektrodenplattform und die Ausbildung als Elektrodenplattform mit integriertem Sensor besonders vorteilhaft sind in Kombination mit der erfindungsgemäßen Mikroperfusionsvorrichtung, kann jede dieser Ausbildungen, insbesondere die Ausbildung einer Elektrode an der Unterseite des Gehäuses, auch bei allen herkömmlichen Mikroperfusionsvorrichtungen verwirklicht werden.

Nachfolgend werden bevorzugte Ausführungsbeispiele der Erfindung anhand von Zeichnungen erläutert. Es zeigen:
- Figur 1: eine Mikroperfusionsvorrichtung nach einem ersten Ausführungsbeispiel mit einem integrierten Sensor und integrierten Elektroden für eine Messeinrichtung,
- Figur 2: die Mikroperfusionsvorrichtung ohne Sensor und Elektroden,
- Figur 3: eine nicht erfindungsgemäße Mikroperfusionsvorrichtung,
- Figur 4: Einstechnadeln mit nicht kreisrunden Außenquerschnitten,
- Figur 5: Perfusionskatheter mit nicht kreisrunden Innenquerschnitten,
- Figur 6: eine Mikroperfusionsvorrichtung mit einer angeschlossenen Messeinrichtung in schematischer Darstellung und
- Figur 7: eine Mikroperfusionsvorrichtung aus dem Stand der Technik.

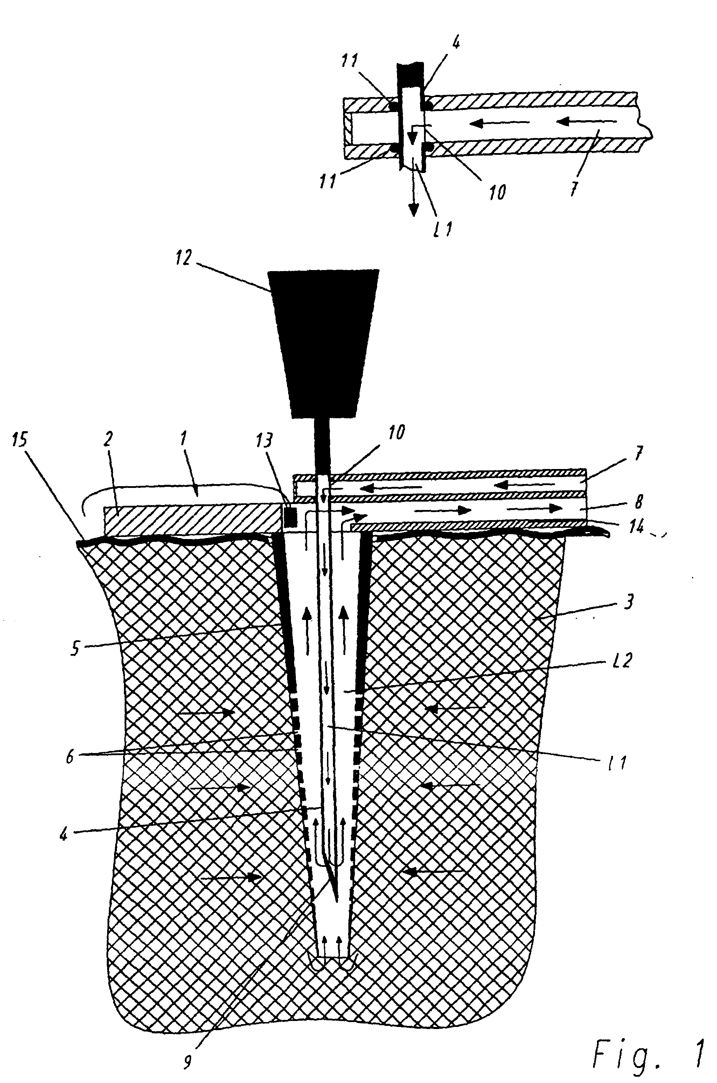

Figur 1 zeigt eine implantierte Mikroperfusionsvorrichtung in einem Längsschnitt. Die Vorrichtung weist ein Gehäuse 1 mit einem Auflageteller 2 auf, an dessen Unterseite ein Klebepatch 15 befestigt ist. Von der Unterseite des Auflagetellers 2 ragt ein flexibler, perforierter Perfusionskatheter 5 senkrecht ab. Der Perfusionskatheter 5 umgibt eine in ihn hineinragende Einstechnadel 4 konzentrisch. Die Einstechnadel 4 ist in der Art von Einstechnadeln ausgebildet, wie sie von Katheterköpfen für die Insulininfusion bekannt sind. Die Einstechnadel 4 wird durch einen schlanken, geraden Hohlzylinder gebildet mit einer vorderen Nadelöffnung 9 am vorderen, distalen Stirnende und einer hinteren Nadelöffnung 10 im Mantel der Einstechnadel 4. Weitere Öffnungen weist die Einstechnadel 4 nicht auf. Zwischen ihren beiden Öffnungen 9 und 10 umschließt die Einstechnadel 4 ein inneres Lumen L1. Zwischen der Einstechnadel 4 und dem Perfusionskatheter 5 ist ein äußeres Lumen L2 in Form eines Ringspalts gebildet.

In dem Gehäuse 1 sind in dem Auflageteller 2 ein Abführkanal 8 und darüberliegend eine Perfusatzuführung in Form eines Zuführkanals 7 ausgebildet. Die Einstechnadel 4 ist in dem Gehäuse 1 geradgeführt in Längsrichtung verschiebbar aufgenommen. Die Geradführung wird von einer Durchgangsbohrung gebildet, die das Gehäuse 1 von einer Oberseite bis zu der gegenüberliegenden Unterseite durchragt. Auf diese Weise durchragt die Einstechnadel 4 sowohl den Zuführkanal 7 als auch den Abführkanal 8. In dem Zuführkanal 7 sind in zwei Ausnehmungen, die je in der Innenwandung des Zuführkanals 7 um die Durchgangsbohrung umlaufen, zwei Dichtungsringe 11 eingesetzt, die die Einstechnadel 4 unter Pressdruck dichtend umgeben. In einer dargestellten, hinteren Verschiebeposition der Einstechnadel 4 kommt die hintere Nadelöffnung 10 zwischen den beiden Dichtungsringen 11 zu liegen. Auf diese Weise wird in der hinteren Verschiebeposition der Einstechnadel 4 eine fluiddichte Verbindung zwischen dem Zuführkanal 7 und dem inneren Lumen L1 und ständig eine fluiddichte Trennung zwischen dem Zuführkanal 7 und dem Abführkanal 8 geschaffen.

Gleichzeitig wird mittels der Dichtungsringe 11 in der hinteren Verschiebeposition der Einstechnadel 4 eine Rastverbindung zwischen der Einstechnadel 4 und dem Gehäuse 1 hergestellt. In der Raststellung, d.h. in der hinteren Verschiebeposition, sind die beiden Dichtungsringe 11 in die hintere Nadelöffnung 10 hineingedrückt. Auf diese Weise wird eine Rast- bzw. Schnappwirkung erzielt. Die hintere Nadelöffnung 10 erstreckt sich in Längsrichtung der Einstechnadel 4 über solch eine Länge, dass beide Dichtungsringe 11 in der hinteren Nadelöffnung 10 zu liegen kommen und je einer der beiden Dichtungsringe 11 an einen hinteren und einen vorderen Öffnungsrand drückt. Für die Bereitstellung der Rastverbindung würde es grundsätzlich genügen, wenn in der hinteren Verschiebeposition nur einer der Dichtungsringe 11 hinter dem hinteren oder den vorderen Rand der hinteren Nadelöffnung 10 zu liegen käme. Das Andrücken sowohl gegen den hinteren als auch gegen den gegenüberliegenden vorderen Öffnungsrand der hinteren Nadelöffnung 10 schafft jedoch eine Rastverbindung, die ein unbeabsichtigtes Verschieben der Einstechnadel 4 in beide Verschieberichtungen verhindert. Die Fluidverbindung zwischen dem Zuführkanal 7 und der Einstechnadel 4 sowie die Rastverbindung zwischen dem Gehäuse 1 und der Einstechnadel 4 sind in Figur 1 als Detail herausgelöst nochmals vergrößert dargestellt.

Zur Erleichterung des manuellen Verschiebens der Einstechnadel 4 ist die Einstechnadel 4 an ihrem hinteren, aus dem Gehäuse 1 herausragenden Ende mit einem Nadelgriffstück 12 versehen.

Figur 1 zeigt die Mikroperfusionsvorrichtung in ihrem Betriebszustand während einer Mikroperfusion, in dem die Einstechnadel 4 sich in dem Gehäuse 1 in ihrer hinteren Verschiebeposition befindet. Vor der Implantation bzw. dem Plazieren des Perfusionskatheters 5 in dem Gewebe 3 durchragt die Einstechnadel 4 in einer vorderen Verschiebeposition den Perfusionskatheter 5. In diesem Ausgangszustand steht die Spitze der Einstechnadel 4 mit der vorderen Nadelöffnung 9 über das vordere Ende des Perfusionskatheters 4 hinaus. Das Nadelgriffstück 12 ist in diesem Ausgangszustand bis gegen die Oberfläche des Gehäuses 1 gedrückt. Zur Plazierung des Perfusionskatheters 5 werden die Einstechnadel 4 und der zumindest an seinem vorderen Ende die Einstechnadel 4 umschmiegende Perfusionskatheter 5 durch die Haut hindurch gestochen und in das Gewebe 3 bis in die in Figur 1 gezeigte Stellung eingeführt. In dieser Stellung liegt der Auflageteller 2 des Gehäuses 1 mit seiner Unterseite flächig auf der Haut auf. Das an der Unterseite des Auflagetellers 2 befestigte Klebepatch 15 bildet eine Klebefläche zur Haut. Durch Andrücken des Gehäuses 1 gegen die Haut wird eine Klebeverbindung hergestellt. Zur Durchführung der Mikroperfusion wird die Einstechnadel 4 nach dem Plazieren und Befestigen des Gehäuses 1 in die in Figur 1 gezeigte hintere Verschiebeposition zurückgezogen. Die Mikroperfusionsvorrichtung ist nun bereit für eine anschließende Mikroperfusion zur Gewinnung des wenigstens einen Inhaltsstoffs der Körperflüssigkeit.

Der Perfusionskatheter 5 ist in einem Mantelbereich zwischen seinem distalen, vorderen Ende und seinem an das Gehäuse 1 angrenzenden, proximalen, hinteren Ende perforiert mit Perforationsöffnungen 6. Vor dem perforierten Bereich verbleibt an dem vorderen Ende des Perfusionskatheters 5 ein nicht perforierter Mantelbereich. Der Perfusionskatheter 4 ist auch im Bereich seines hinteren Endes nicht perforiert. Nach vorn ist der Perfusionskatheter 4 stirnseitig offen. Bei einem Spülen des Perfusionskatheters 5 entsteht eine sogenannte Open-Flow-Microperfusion. Ein Perfusat wird durch einen angeschlossenen Zuführkatheter in den Zuführkanal 7 des Gehäuses 1 geführt, tritt durch die hintere Nadelöffnung 10 in die hohle Einstechnadel 4 ein, durchströmt die Einstechnadel 4 und tritt durch die distale, vordere Nadelöffnung 9 an der Nadelspitze in den Perfusionskatheter 5 aus. Nach dem Austritt strömt das Perfusat in dem äußeren Lumen L2 zwischen dem Außenmantel der Einstechnadel 4 und dem Perfusionskatheter 5 zurück in Richtung auf das Gehäuse 1. Bei dem Zurückströmen werden aufgrund einer sich im äußeren Lumen L2 ergebenden Düsenwirkung Körperflüssigkeit F und aufgrund eines Konzentrationsgefälles zwischen der Körperflüssigkeit F und dem Perfusat selektiv der zu gewinnende Inhaltsstoff der Körperflüssigkeit oder selektiv mehrere Inhaltsstoffe der Körperflüssigkeit durch die Perforationsöffnungen 6 eingesogen und im Rückstrom des Perfusats mitgenommen. Das zurückströmende Perfusat tritt durch eine in dem Gehäuse 1 ausgebildete Fluidverbindung aus dem äußeren Lumen L2 in den Abführkanal 8 und strömt anschließend durch einen an den Abführkanal 8 angeschlossenen Abführkatheter in einen Sammelbehälter ab. Der Perfusionskatheter 5 ist mit dem Gehäuse 1 fluiddicht verbunden.

In dem Gehäuse 1 ist in einem Strömungsquerschnitt des zurückströmenden Perfusats ein Miniatursensor 13 angeordnet. Der Sensor 13 ist in dem Abführkanal 8 im Gehäuse 1 angeordnet. Vorzugsweise ist er in einem Strömungsquerschnitt unmittelbar stromabwärts von dem Perfusionskatheter 5 angeordnet. Der Sensor 13 wird jedoch nicht implantiert, sondern befindet sich in einem Strömungsquerschnitt außerhalb des Gewebes 3. Vorzugsweise kann er auch noch nachträglich, d.h. nach dem Plazieren der Mikroperfusionsvorrichtung in das Gehäuse 1 eingesetzt, beispielsweise eingeklippt werden.

Die Mikroperfusionsvorrichtung dient nicht nur als Sensorplattform, sondern gleichzeitig auch als Elektrodenplattform für eine Messeinrichtung. Eine Arbeitselektrode 14 ist in dem Gehäuse 1 an einer Innenwandung des Abführkanals 8 ausgebildet oder bildet einen Bereich der Innenwandung. Das Klebepatch 15 ist selbst elektrisch leitend und ist elektrisch leitend mit der Haut verbunden. Es dient der Messeinrichtung als Gegenelektrode zur Arbeitselektrode 15. Vorzugsweise ist die Auflagefläche des Klebepatch 15 so groß, dass es gleichzeitig auch noch eine Referenzelektrode bildet.

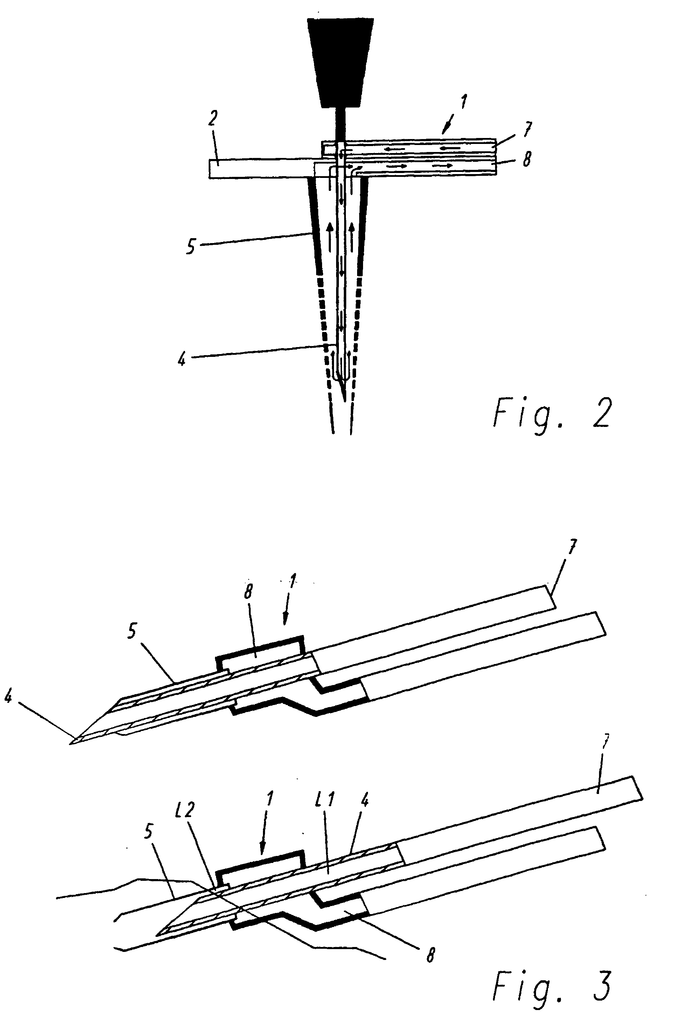

Figur 2 zeigt die gleiche Mikroperfusionsvorrichtung wie Figur 1. Die Vorrichtung weist einen integrierten Sensor und integrierte Elektroden jedoch nicht auf. Ansonsten entspricht sie der Mikroperfusionsvorrichtung des ersten Ausführungsbeispiels identisch.

Die Stelle, an der in dem Abführkanal 8 im ersten Ausführungsbeispiel der Sensor 13 angeordnet ist, bleibt im Abführkanal 8 dieser Vorrichtung einfach frei. Der Sensor und die Elektroden der Messeinrichtung sind bei Verwendung der Vorrichtung nach Figur 2 in bekannter Weise anderweitig anzuordnen.

Figur 3 zeigt eine Mikroperfusionsvorrichtung, die sich von den erfindungsgemäßen Mikroperfusionsvorrichtungen der beiden anderen Beispiele dadurch unterscheidet, dass in dem Gehäuse 1 ein Zuführkanal für das Perfusat nicht ausgebildet ist. Eine Perfusatzuführung 7 wird durch einen unmittelbar und permanent mit der Einstechnadel 4 verbundenen Zuführkatheter 7 gebildet. Die Einstechnadel 4 steht über ihre hintere Nadelöffnung 10 in einer permanenten Fluidverbindung mit dem Zuführkatheter 7. Die Geradführung der Einstechnadel 4 in dem Gehäuse 1 und die Zufuhr des Perfusats erfolgen somit getrennt voneinander. Der Zuführkatheter 7 wird bei dem Zurückziehen der Einstechnadel 4 in die hintere Verschiebeposition mit der Einstechnadel 4 mitbewegt.

Die obere Abbildung der Figur 3 zeigt die Mikroperfusionsvorrichtung in ihrem Ausgangszustand vor der Plazierung in dem Gewebe, in dem die Einstechnadel 4 ihre vordere Verschiebeposition in Bezug auf das Gehäuse 1 und den Perfusionskatheter 5 einnimmt. In diesem Zustand befindet sich die Mikroperfusionsvorrichtung auch noch nach der Plazierung in dem Gewebe, bis die Einstechnadel 4 ein Stück weit bis in ihre hintere Verschiebeposition zurückgezogen wird. In einem Strömungsquerschnitt des Zuführkatheters ist ein Absperrventil oder ein Absperrschieber angeordnet, damit der Zufluss von Perfusat zur Einstechnadel 4 bis zur Perfusion unterbrochen werden kann.

Die untere Abbildung der Figur 3 zeigt die Einstechnadel 4 in ihrer hinteren Verschiebeposition. Durch das Zurückziehen bzw. Zurückverschieben der Einstechnadel 4 ein Stück weit hinter das vordere Ende des Perfusionskatheters 5 wird ein für die anschließende Perfusion geeignetes, koaxiales Strömungssystem geschaffen mit dem von der Einstechnadel 4 umschlossenen Lumen L1 und dem zwischen der Einstechnadel 4 und dem Perfusionskatheter 5 als Ringraum gebildeten äußeren Lumen L2. Das äußere Lumen L2 steht in Fluidverbindung mit dem wieder im Gehäuse 1 ausgebildeten Abführkanal 8, an dem ein Abführkatheter angeschlossen ist. In Bezug auf die weiteren Konstruktionsmerkmale und Funktionsweise gelten die Ausführungen zum ersten Ausführungsbeispiel.

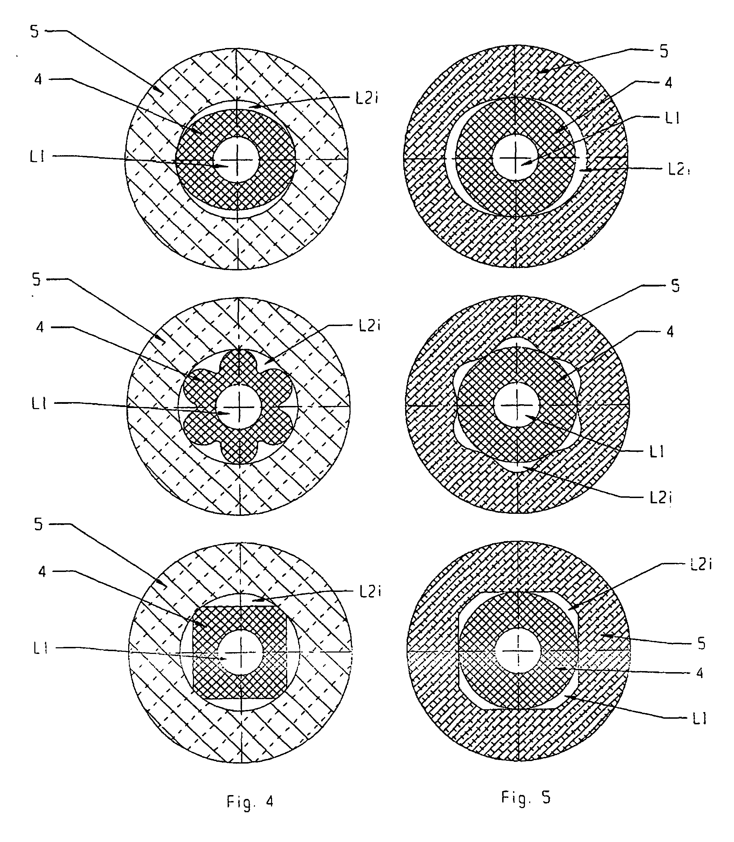

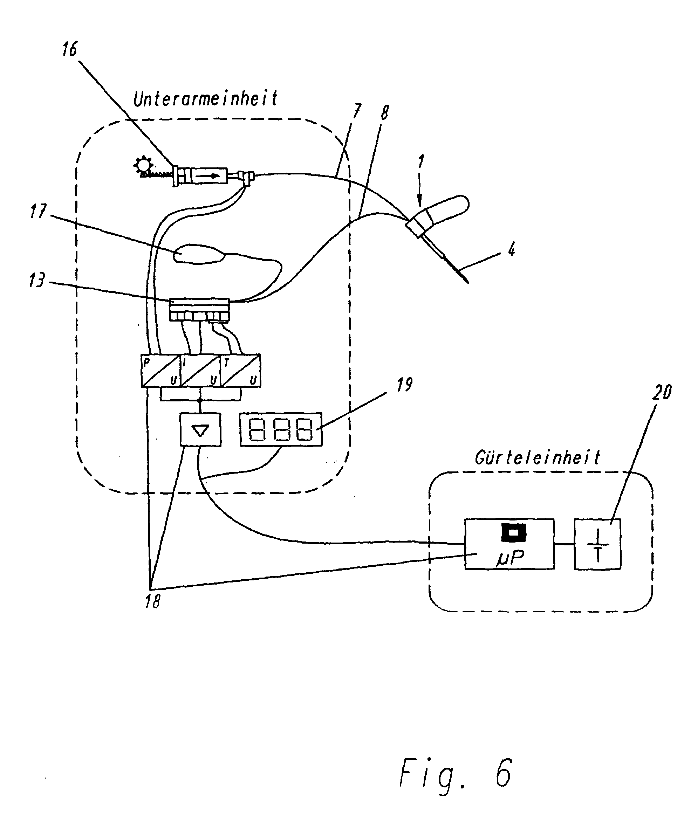

Die Figuren 4 und 5 zeigen Kombinationen von Einstechnadeln 4 und Perfusionskathetern 5, deren Querschnittsformen jeweils derart aufeinander angepasst sind, dass in dem äußeren Lumen L2 über die gesamte Strömungslänge des zurückströmenden Fluids stets ein ausreichender Strömungsquerschnitt verbleibt und dennoch der Perfusionskatheter 5 die Einstechnadel 4 eng anliegend umgibt bzw. umschmiegt. In den Querschnittskombinationen der Figur 4 ist jeweils der Innenquerschnitt des Perfusionskatheters 5 im neutralen, ungespannten Zustand kreisrund, während der Außenquerschnitt der Einstechnadel 4 von der kreisrunden Querschnittsform abweicht. In den Querschnittskombinationen der Figur 5 ist der Außenquerschnitt der Einstechnadel 4 hingegen kreisrund, und es weicht der Innenquerschnitt des Perfusionskatheters 5 von der kreisrunden Form ab. Im Einbauzustand ist der Perfusionskatheter 5 auch im neutralen Zustand um die Einstechnadel herum gespannt. Zwischen den Andruckstellen der Perfusionskatheters 5 an die Einstechnadel 4 werden auf diese Weise längs der Einstechnadel 4 und um die Einstechnadel 4 herum verteilt Teillumen L2i gebildet, durch die hindurch das Perfusat zurückströmt. Indem der Außenquerschnitt der Einstechnadel 4 und der Innequerschnitt des Perfusionskatheters 5 so ausgebildet sind, dass der Perfusionskatheter 5 lediglich in Längsstreifen an die Einstechnadel 4 drückt und zwischen den Andruckstreifen Teillumen L2i verbleiben, kann der Perfusionskatheter 5 über seine gesamte implantierte Länge oder zumindest über eine vordere Teillänge um die Einstechnadel 4 herum gespannt sein. Die Einstechnadel 4 stützt somit den Perfusionskatheter 5, was bei dem Durchstechen der Haut und dem weiteren Einführen in das Gewebe von Vorteil ist.

Figur 6 zeigt eine Perfusionsvorrichtung mit einer angeschlossenen Mess- und Auswerteeinrichtung. An die nur schematisch dargestellte Mikroperfusionsvorrichtung, gebildet beispielsweise durch die Vorrichtung der Figur 2 oder der Figur 3 sind ein Zuführkatheter 7 und ein Abführkatheter 8 angeschlossen. Eine Mikropumpe 16 fördert ein Perfusat durch den Zuführkatheter 7 hindurch in die Einstechnadel 4. Das zurückströmende Perfusat wird dem externen Sensor 13 zugeführt und anschließend in einen Sammelbehälter 17 geleitet und entsorgt. Das zu dem Sensor 13 zurückströmende Perfusat enthält den wenigstens einen Inhaltsstoff, dessen Konzentration durch Messung ermittelt werden soll. Wird dieser Inhaltsstoff durch Glukose gebildet, so ist der Sensor 13 ein Glukosesensor. Die Messsignale des Sensors 13 werden einer Auswerteeinrichtung 18 zugeführt, die daraus die Konzentration des wenigstens einen Inhaltsstoffs ermittelt und auf einem Display 19 zur Anzeige bringt. Im Ausführungsbeispiel der Figur 6 wird die Mikroperfusionsvorrichtung im Unterarmgewebe plaziert. Die Mikropumpe 16, der Sensor 13, der Sammelbehälter 17, ein Teil der Auswerteeinrichtung 18 und das Display 19 können in einer Unterarmeinheit zusammengefasst untergebracht sein. Ein Mikroprozessor der Auswerteeinrichtung 18 und eine Batterie 20 können zusammen in einer Gürteleinheit untergebracht und mit der Unterarmeinheit für den Austausch von Daten und zur Versorgung der Unterarmeinheit mit Energie verbunden sein. Die Auswerteeinheit 18, insbesondere der Mikroprozessor, übernimmt nicht nur die Auswertung der Messsignale vom Sensor 13, sondern auch die Steuerung der Mikropumpe 16.

Bei Verwendung der besonders bevorzugten Mikroperfusionsvorrichtung des ersten Ausführungsbeispiels ist der Sensor 13 nicht in einer externen Unterarmeinheit, sondern in dem Gehäuse 1 der Vorrichtung integriert angeordnet.

### Bezugszeichen

- 1: Gehäuse
- 2: Auflageteller
- 3: Gewebe
- 4, 4a: Einstechnadel
- 5, 5a: Perfusionskatheter
- 6: Perforationsöffnungen
- 7, 7a: Zuführung, Zuführkanal, Zuführkatheter
- 8: Abführkanal
- 9: vordere Nadelöffnung
- 10: hintere Nadelöffnung
- 11: Dichtungsring
- 12: Nadelgriffstück
- 13: Sensor
- 14: Arbeitselektrode
- 15: Klebepatch, Gegenelektrode, Referenzelektrode
- 16: Mikropumpe
- 17: Sammelbehälter
- 18: Auswerteeinrichtung
- 19: Display
- 20: Batterie

## Patentansprüche

1. Mikroperfusionsvorrichtung zur Gewinnung wenigstens eines Inhaltsstoffs einer Körperflüssigkeit durch einen subkutan plazierten Perfusionskatheter (5), die Vorrichtung umfassend:
a) ein Gehäuse (1),
b) eine Perfusatzuführung (7),
c) eine hohle Einstechnadel (4), die das Gehäuse (1) durchragt und eine vordere Nadelöffnung (9) und eine hintere Nadelöffnung (10) aufweist,
d) den Perfusionskatheter (5), der von dem Gehäuse (1) abragt und die Einstechnadel (4) umgibt,
e) und einen in dem Gehäuse (1) gebildeten Abführkanal (8), der zur Abführung des Perfusats mit dem Perfusionskatheter (5) verbunden ist,
f) wobei die Einstechnadel (4) aus einer vorderen Verschiebeposition, in der sie den Perfusionskatheter (5) durchragt, in eine hintere Verschiebeposition, in der ihre vordere Nadelöffnung (9) in den Perfusionskatheter (5) hineinragt und von dem Perfusionskatheter (5) überragt wird, verschiebbar von dem Gehäuse (1) aufgenommen ist
g) und die hintere Nadelöffnung (10) zumindest in der hinteren Verschiebeposition der Einstechnadel (4) eine Fluidverbindung von der Perfusatzuführung (7) in die Einstechnadel (4) bildet,
wobei die Vorrichtung **dadurch gekennzeichnet ist,**
**dass** die hintere Nadelöffnung (10) in einer Mantelfläche der Einstechnadel (4) gebildet ist
und **dass** ein Dichtungsring (11) die Einstechnadel (4) unter Pressung umgibt und in der hinteren Verschiebeposition der Einstechnadel (4) in die hintere Nadelöffnung (10) vorschnappt.

2. Mikroperfusionsvorrichtung nach Anspruch 1, **dadurch gekennzeichnet, dass** der Perfusionskatheter (5) einen für den wenigstens einen Inhaltsstoff durchlässigen Kathetermantel aufweist.

3. Mikroperfusionsvorrichtung nach dem vorhergehenden Anspruch, **dadurch gekennzeichnet, dass** Perforationsöffnungen (6) des Perfusionskatheters (5) in Längsrichtung des Perfusionskatheters (5) länger als in Umfangsrichtung des Perfusionskatheters (5) sind.

4. Mikroperfusionsvorrichtung nach einem der vorhergehenden Ansprüche, **dadurch gekennzeichnet, dass** eine Querschnittsform der Einstechnadel (4) und eine Querschnittsform des Perfusionskatheters (5) auf einer Länge, auf welcher der Perfusionskatheter (5) an der Einstechnadel (4) anliegt, voneinander abweichen, so dass zwischen der Einstechnadel (4) und dem Perfusionskatheter (5) ein Strömungsquerschnitt für das Perfusat verbleibt.

5. Mikroperfusionsvorrichtung nach einem der vorhergehenden Ansprüche, **dadurch gekennzeichnet, dass** die Einstechnadel (4) und/oder der Perfusionskatheter (5) auf einer Länge, auf welcher der Perfusionskatheter (5) an der Einstechnadel (4) anliegt, eine äußere Mantelfläche aufweist, die im Querschnitt gesehen nicht kreisrund ist.

6. Mikroperfusionsvorrichtung nach einem der vorhergehenden Ansprüche, **dadurch gekennzeichnet, dass** die Einstechnadel (4) in ihrer hinteren Verschiebeposition an dem Gehäuse (1) festgelegt ist.

7. Mikroperfusionsvorrichtung nach einem der vorhergehenden Ansprüche, **dadurch gekennzeichnet, dass** die Einstechnadel (4) an dem Gehäuse (1) in ihrer hinteren Verschiebeposition mittels einer Rastverbindung festgelegt ist.

8. Mikroperfusionsvorrichtung nach einem der vorhergehenden Ansprüche, **dadurch gekennzeichnet, dass** ein Sensor (13) einer Messeinrichtung zur Messung der Konzentration des wenigstens einen Inhaltsstoffs der Körperflüssigkeit in oder an dem Gehäuse (1) in dem Abführkanal (8) angeordnet ist.

9. Mikroperfusionsvorrichtung nach einem der vorhergehenden Ansprüche, **dadurch gekennzeichnet,**
**dass** eine Messeinrichtung vorgesehen ist, welche zur Messung der Konzentration des wenigstens einen Inhaltsstoffs der Körperflüssigkeit eine Arbeitselektrode (14) und eine Gegenelektrode (15) aufweist
und **dass** die Gegenelektrode (15) an einer Unterseite des Gehäuses ( 1 ), die nach der Plazierung der Vorrichtung mit einer Gewebeoberfläche in Kontakt steht, ausgebildet ist.

10. Mikroperfusionsvorrichtung nach einem der vorhergehenden Ansprüche, **dadurch gekennzeichnet,**
**dass** eine Messeinrichtung vorgesehen ist, welche zur Messung der Konzentration des wenigstens einen Inhaltsstoffs der Körperflüssigkeit eine Arbeitselektrode (14) und eine Gegenelektrode (15) aufweist
und **dass** die Arbeitselektrode (14) in dem Abführkanal (8) ausgebildet ist.

## Claims

1. A micro perfusion device for obtaining at least one constituent of a body fluid using a subcutaneously positioned perfusion catheter (5), said device comprising:
a) a casing (1);
b) a perfusate supply (7);
c) a hollow injection needle (4) which protrudes through said casing (1) and comprises a front needle opening (9) and a rear needle opening (10);
d) said perfusion catheter (5) which projects from said casing (1) and surrounds said injection needle (4);
e) and a discharge channel (8) formed in said casing (1), said discharge channel being connected to said perfusion catheter (5) for discharging said perfusate;
f) wherein said injection needle (4) is accommodated by said casing (1) such that it may slide from a front sliding position in which it protrudes through said perfusion catheter (5) to a rear sliding position in which its front needle opening (9) protrudes into said perfusion catheter (5) and said perfusion catheter (5) protrudes beyond said front needle opening (9);
g) and wherein said rear needle opening (10) forms a fluid connection from said perfusate supply (7) to said injection needle (4) at least in said rear sliding position of said injection needle (4),
wherein said device is **characterised in that**
said rear needle opening (10) is formed in a surface area of said injection needle (4) and **in that** a sealing ring (11) surrounds and presses said injection needle (4), and latches into said rear needle opening (10) in said rear sliding position of said injection needle (4).

2. The micro perfusion device as set forth in claim 1, **characterised in that** said perfusion catheter (5) comprises a catheter surface permeable to said at least one constituent.

3. The micro perfusion device as set forth in the preceding claim, **characterised in that** perforation openings (6) of said perfusion catheter (5) are longer in the longitudinal direction of said perfusion catheter (5) than in the circumferential direction of said perfusion catheter (5).

4. The micro perfusion device as set forth in any one of the preceding claims, **characterised in that** a cross-sectional shape of said injection needle (4) and a cross-sectional shape of said perfusion catheter (5) deviate from each other over a length along which said perfusion catheter (5) abuts said injection needle (4), such that a flow cross-section for said perfusate remains between said injection needle (4) and said perfusion catheter (5).

5. The micro perfusion device as set forth in any one of the preceding claims, **characterised in that** said injection needle (4) and/or said perfusion catheter (5) comprises an outer surface area which is not circular when seen in cross-section, over a length along which said perfusion catheter (5) abuts said injection needle (4).

6. The micro perfusion device as set forth in any one of the preceding claims, **characterised in that** said injection needle (4) is fixed to said casing (1) in its rear sliding position.

7. The micro perfusion device as set forth in any one of the preceding claims, **characterised in that** said injection needle (4) is fixed to said casing (1) in its rear sliding position by means of a locking connection.

8. The micro perfusion device as set forth in any one of the preceding claims, **characterised in that** a sensor (13) of a measuring means for measuring the concentration of said at least one constituent of said body fluid is arranged in or on said casing (1), in said discharge channel (8).

9. The micro perfusion device as set forth in any one of the preceding claims, **characterised in that**
a measuring means is provided for measuring the concentration of said at least one constituent of said body fluid, which comprises a working electrode (14) and a counter electrode (15)
and **in that** said counter electrode (15) is formed on a lower side of said casing (1), said lower side contacting a tissue surface once said device has been positioned.

10. The micro perfusion device as set forth in any one of the preceding claims, **characterised in that**
a measuring means is provided for measuring the concentration of said at least one constituent of said body fluid, which comprises a working electrode (14) and a counter electrode (15)
and **in that** said working electrode (14) is formed in said discharge channel (8).

## Revendications

1. Dispositif de microperfusion pour l'obtention d'au moins un constituant d'un liquide corporel par un cathéter de perfusion (5) placé en sous-cutané, le dispositif comprenant :
a) un boîtier (1)
b) une canule d'amenée du perfusat (7),
c) une aiguille creuse (4) qui traverse le boîtier (1) et présente une lumière d'aiguille avant (9) et une lumière d'aiguille arrière (10),
d) le cathéter à perfusion (5) qui part du boîtier (1) et entoure l'aiguille (4),
e) et une canule d'évacuation (8) formée dans le boîtier (1) reliée au cathéter de perfusion (5) pour évacuer le perfusat,
f) l'aiguille (4) étant accueillie par le boîtier (1) de manière coulissante depuis une position coulissante avant, dans laquelle elle traverse le cathéter de perfusion (5) vers une position coulissante arrière, dans laquelle sa lumière avant (9) pénètre dans le cathéter de perfusion (5) et est dominée par le cathéter de perfusion (5)
g) et la lumière d'aiguille arrière (10) formant, du moins dans la position coulissante arrière de l'aiguille (4), une liaison fluide depuis la canule d'amenée du perfusat (7) dans l'aiguille (4),
le dispositif étant **caractérisé en ce que**
la lumière d'aiguille arrière (10) est formée dans une surface de l'enveloppe de l'aiguille (4)
et **en ce qu'**une bague d'étanchéité (11) entoure l'aiguille (4) sous pression et saisit la lumière d'aiguille arrière (10) lorsque l'aiguille (4) est en position coulissante arrière.

2. Dispositif de microperfusion selon revendication 1, **caractérisé en ce que** le cathéter de perfusion (5) présente une enveloppe perméable pour le au moins un constituant.

3. Dispositif de microperfusion selon la revendication précédente, **caractérisé en ce que** les lumières de perforation (6) du cathéter de perfusion (5) sont plus longues, dans le sens longitudinal du cathéter de perfusion (5) que dans le sens périphérique du cathéter de perfusion (5).

4. Dispositif de microperfusion selon l'une des revendications précédentes, **caractérisé en ce qu'**une forme de section de l'aiguille (4) et une forme de section du cathéter de perfusion (5) sont différentes l'une de l'autre, sur une longueur sur laquelle le cathéter de perfusion (5) plaque contre l'aiguille (4), de telle manière qu'entre l'aiguille (4) et le cathéter de perfusion (5) il reste une section d'écoulement pour le perfusat.

5. Dispositif de microperfusion selon l'une des revendications précédentes, **caractérisé en ce que** l'aiguille (4) et/ou le cathéter de perfusion (5) présente, sur une longueur sur laquelle le cathéter de perfusion (5) plaque contre l'aiguille (4), une surface d'enveloppe extérieure qui, du point de vue de la section, n'est pas circulaire.

6. Dispositif de microperfusion selon l'une des revendications précédentes, **caractérisé en ce que** l'aiguille (4) est immobilisée sur le boîtier (1) dans sa position coulissante arrière.

7. Dispositif de microperfusion selon l'une des revendications précédentes, **caractérisé en ce que** l'aiguille (4) est immobilisée sur le boîtier (1) dans sa position coulissante arrière au moyen d'un connecteur à encoches.

8. Dispositif de microperfusion selon l'une des revendications précédentes, **caractérisé en ce qu'**un capteur (13) d'un dispositif de mesure est placé dans ou sur le boîtier (1) dans la canule d'évacuation (8) pour mesurer la concentration du au moins un constituant du liquide corporel.

9. Dispositif de microperfusion selon l'une des revendications précédentes, **caractérisé en ce que**,
un dispositif de mesure est prévu qui présente une électrode de travail (14) et une contre-électrode (15) pour mesurer la concentration du au moins un constituant du liquide corporel,
et **en ce que** la contre-électrode (15) est formée sur un côté inférieur du boîtier (1) en contact avec une surface cutanée après la mise en place du dispositif.

10. Dispositif de microperfusion selon l'une des revendications précédentes, **caractérisé en ce que**
un dispositif de mesure est prévu qui présente une électrode de travail (14) et une contre-électrode (15) pour mesurer la concentration du au moins un constituant du liquide corporel
et **en ce que** l'électrode de travail (14) est formée dans la canule d'évacuation (8).
